(19) Europäisches Patentamt
European Patent Office
Office européen des brevets

(11) Veröffentlichungsnummer: **0 007 019**
**A1**

(12) # EUROPÄISCHE PATENTANMELDUNG

(21) Anmeldenummer: 79102022.5

(22) Anmeldetag: 19.06.79

(51) Int. Cl.³: C 07 D 231/20
A 61 K 31/415, C 07 D 231/22

(30) Priorität: 29.06.78 DE 2828529

(43) Veröffentlichungstag der Anmeldung:
23.01.80 Patentblatt 80/2

(84) Benannte Vertragsstaaten:
AT BE CH DE FR GB IT LU NL SE

(71) Anmelder: Kali-Chemie Pharma GmbH
Hans-Böckler-Allee 20 Postfach 220
D-3000 Hannover 1(DE)

(72) Erfinder: Heinemann, Henning, Dipl.-Chem., Dr. rer.nat
Bergiusstrasse 18
D-3000 Hannover(DE)

(72) Erfinder: Milkowski, Wolfgang, Dipl.-Chem., Dr.rer.nat.
Fasanenweg 13
D-3167 Burgdorf(DE)

(72) Erfinder: Zeugner, Horst, Dipl.-Chem., Dr.rer.nat.
Havelweg 10
D-3000 Hannover 73(DE)

(72) Erfinder: Hempel, Reinhard, Dr.med.
Wolfsburger Damm 32
D-3000 Hannover 61(DE)

(72) Erfinder: Weiser, Dieter, Dr.rer.nat.
Sextrostrasse 23
D-3000 Hannover(DE)

(74) Vertreter: Lauer, Dieter, Dr. et al,
c/o Kali-Chemie Aktiengesellschaft Postfach 220
D-3000 Hannover 1(DE)

(54) **Neue 5-Phenylpyrazol-Derivate und ihre Salze; Verfahren zu deren Herstellung und diese Verbindungen enthaltende Arzneimittel.**

(57) Neue 5-Phenylpyrazol-Derivate werden offenbart, welche die Formel

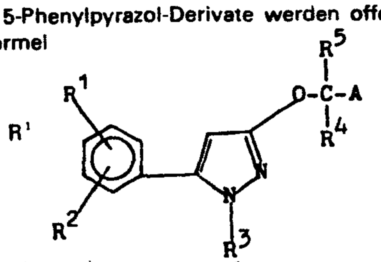

R⁵
O–C–A
R⁴

besitzen, worin $R^1$ und $R^2$ ein Wasserstoff- oder Chloratom oder eine Trifluormethylgruppe, $R^3$ ein Wasserstoffatom einen Phenyl-, Benzyl- oder Niederalkylrest, $R^4$ ein Wasserstoffatom oder einen Methylrest, $R^5$ einen Niederalkylrest und A Carboxyl, Alkoxycarbonyl, Cyano und gegebenenfalls substituiertes Aminocarbonyl bedeuten. Die Verbindungen zeichnen sich durch gute lipidsenkende Eigenschaften aus. Sie eignen sich als Langzeittherapeutika, da Veränderungen der Leber nicht auftreten. Als Ausgangsmaterialien für die Herstellung dieser Verbindungen eignen sich die entsprechenden 3-Phenylpyrazolin-5-one.

EP 0 007 019 A1

Kali-Chemie Pharma GmbH

Hannover, 26.6.1978
Z1-PA/Dr.Ha/Ka

## Patentanmeldung

### Neue 5-Phenylpyrazol-Derivate und ihre Salze; Verfahren zu deren Herstellung und diese Verbindungen enthaltende Arzneimittel

Die Erfindung betrifft neue 5-Phenylpyrazol-Derivate, deren pharmakologisch verträgliche Salze, Verfahren zu deren Herstellung sowie diese Verbindungen enthaltende pharmazeutische Zusammensetzungen.

Der vorliegenden Erfindung liegt die Aufgabe zugrunde, neue 5-Phenylpyrazol-Derivate mit wertvollen pharmakologischen und therapeutischen Eigenschaften zu schaffen.

Überraschenderweise wurde nämlich gefunden, daß die neuen Verbindungen einen blutlipidsenkenden Effekt besitzen, ohne eine vermehrte Fetteinlagerung in die Leberzellen und ohne eine Erhöhung des Lebergewichts zu bewirken.

Darüberhinaus besitzen diese Verbindungen antiallergische Eigenschaften, insbesondere bei peroraler Verabreichung.

Die neuen 5-Phenylpyrazol-Derivate besitzen die Formel I

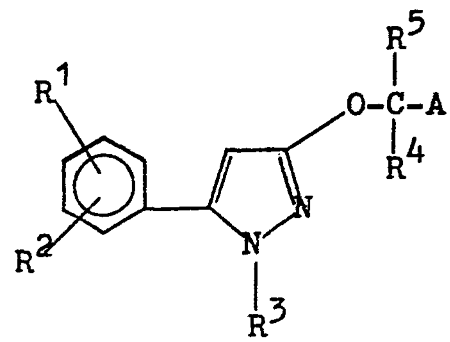

worin $R^1$ und $R^2$ gleich und verschieden sein können und ein Wasserstoff- oder Chloratom oder eine Trifluormethylgruppe bedeuten, $R^3$ ein Wasserstoffatom, ein Phenyl-, Benzyl- oder Alkylrest mit 1 bis 6 C-Atomen, welcher geradkettig, verzweigt oder cyclisch sein kann, $R^4$ ein Wasserstoffatom oder Methylrest, $R^5$ ein Alkylrest mit 1 bis 4 C-Atomen, geradkettig oder verzweigt, und A Carboxyl, Alkoxycarbonyl, worin der Alkoxyrest 1 bis 4 C-Atome hat und geradkettig oder verzweigt ist, Cyano, Aminocarbonyl, Monoalkylaminocarbonyl oder Dialkylaminocarbonyl mit Alkylresten mit 1 bis 3 C-Atomen ist sowie die pharmakologisch verträglichen Salze der Säuren.

Als niedermolekulare Alkylreste für $R^3$ kommen Alkylreste mit 1 bis 6 C-Atomen, welche geradkettig oder verzweigt sind, in Frage, so Methyl, Äthyl, Propyl, Isopropyl, Butyl, 2-Butyl, 2-Methylpropyl, tert.-Butyl, Pentyl, 2-Pentyl, 2-Methylbutyl, 3-Methylbutyl, 3-Pentyl, 1,1-Dimethylpropyl, Neopentyl, Hexyl, 2-Hexyl, 1,1-Dimethylbutyl. Besonders bevorzugt werden Methyl, Äthyl, Propyl, Isopropyl, Butyl oder Isobutyl. Als Cycloalkylreste eignen sich vorzugsweise Cyclopentyl und Cyclohexyl. Als $R^5$ eignen sich die Alkylreste mit 1 bis 4 C-Atomen, wobei Methyl, Äthyl, Propyl und Isopropyl bevorzugt werden.

Geeignete Alkoxyreste sind Methoxy, Äthoxy, Propoxy, Isopropoxy, Butoxy oder Isobutoxy. Bevorzugt sind die Methoxy- und Äthoxyreste. Im Dialkylaminocarbonylrest können die geradkettigen oder verzweigten Alkylreste gleich oder verschieden sein. Gegenstand der Erfindung sind ferner Verfahren zur Herstellung der Verbindungen der allgemeinen Formel I.

Die neuen Verbindungen werden dadurch erhalten, daß man

a) ein Alkalimetallsalz des 3-Phenylpyrazolin-5-on der Formel II

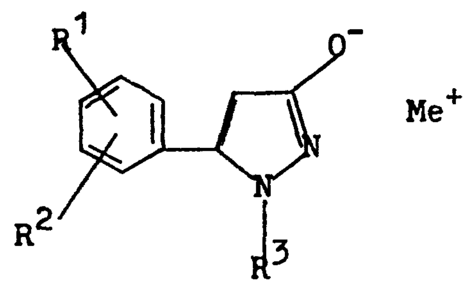

worin $R^1$, $R^2$ und $R^3$ die obengenannte Bedeutung haben und $Me^+$ ein Alkalimetallion ist, mit einer Verbindung der Formel III

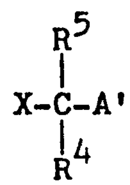

worin $R^4$ und $R^5$ die obengenannte Bedeutung haben, A' eine Alkoxycarbonyl- eine Cyano- oder eine ggf. substituierte Aminocarbonylgruppe und X ein Halogenatom, vorzugsweise Chlor oder Brom, ist, zu einer Verbindung der Formel I umsetzt, worin $R^1$, $R^2$, $R^3$, $R^4$ und $R^5$ die obengenannte Bedeutung haben und A eine Cyano-, eine Alkoxycarbonyl- oder eine ggf. substituierte Aminocarbonylgruppe ist, gewünschtenfalls ein Säurederivat zur freien Säure oder zu deren physiologisch verträglichem Salz hydrolysiert,

oder daß man

b) ein Alkalimetallsalz des 3-Phenylpyrazolin-5-on der Formel II

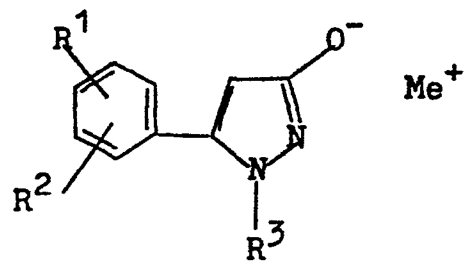

worin $R^1$, $R^2$ und $R^3$ die obengenannte Bedeutung haben und $Me^+$ ein Alkalimetallion ist, mit einem Haloform und einer Carbonylverbindung der Formel IV

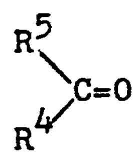

worin $R^4$ und $R^5$ die obengenannte Bedeutung haben, in Gegenwart eines Alkalihydroxids zu einem Alkalisalz einer Verbindung der Formel I umsetzt, in welcher $R^1$, $R^2$, $R^3$, $R^4$ und $R^5$ die obengenannte Bedeutung haben und A eine Carboxylgruppe ist und ggf. das Alkalimetallsalz zur freien Säure hydrolysiert, oder daß man

c) eine Säure der Formel V

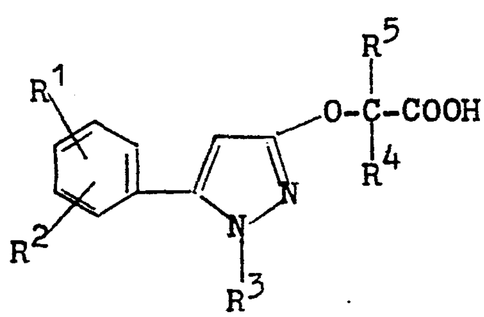

in welcher $R^1$, $R^2$, $R^3$, $R^4$ und $R^5$ die obengenannte Bedeutung haben,

$c_1$) in das entsprechende Amid umwandelt, dessen Amidfunktion durch Alkyl mono- oder disubstituiert sein kann, oder

$c_2$) in den entsprechenden Ester der Formel I umwandelt, oder daß man

d) ein Nitril der Formel VI

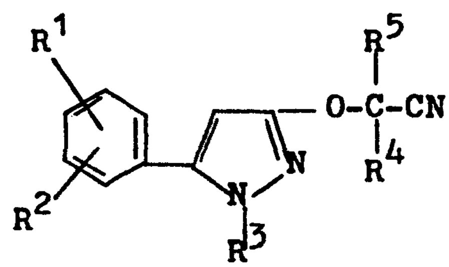

in welcher $R^1$, $R^2$, $R^3$, $R^4$ und $R^5$ die obengenannte Bedeutung haben, in das entsprechende Amid umwandelt oder daß man

e) einen Ester der Formel VII

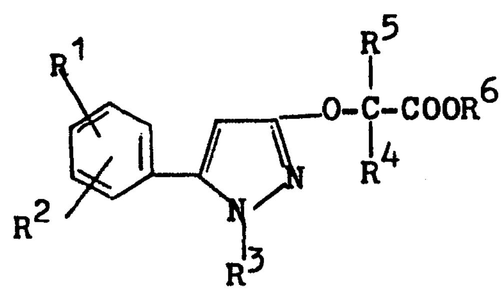

worin $R^1$, $R^2$, $R^3$, $R^4$ und $R^5$ die obengenannte Bedeutung haben und $R^6$ eine niedere Alkylgruppe ist, in das entsprechende Amid umwandelt, dessen Amidfunktion durch Alkyl mono- oder disubstituiert sein kann, oder daß man

f) einen Ester der Formel VII, worin $R^1$, $R^2$, $R^4$, $R^5$ und $R^6$ die obengenannte Bedeutung haben und $R^3$ ein Wasserstoffatom ist, in den entsprechenden Ester umwandelt, in welchem $R^3$ Alkyl, Cycloalkyl oder Benzyl ist.

Der vorstehend unter a) definierte Verfahrensaspekt des erfindungsgemäßen Verfahrens stellt eine an sich bekannte Alkylierung am Sauerstoffatom in 5-Stellung des Pyrazolinons dar. Vorteilhafterweise wird dabei ein Alkalimetallsalz des 3-Phenylpyrazolin-5-ons mit dem Halogenid der Formel III zur Reaktion gebracht. Zweckmäßigerweise führt man die Umsetzung in einem inerten Lösungsmittel, z.B. Dimethylformamid, 1,4-Dioxan, Dimethylsulfoxid oder Toluol, zwischen 0°C und dem Siedepunkt des Lösungsmittels, vorzugsweise zwischen 60 und 80°C durch. Im Falle des Einsatzes des nichtmetallierten Pyrazolinons wird in Gegenwart eines basischen Katalysators, wie Natriumhydroxid, Kaliumhydroxid, Natriummethylat, Natriumäthylat, Kalium-tert.-butylat, gearbeitet. Als inerte Lösungsmittel eignen sich Dimethylformamid, Toluol oder niedermolekulare Alkohole, allein oder im Gemisch mit Wasser. Man kann aber auch Alkalimetallhydride, vorzugsweise Natriumhydrid, Alkaliamide, wie Natriumamid oder Lithiumdiisopropylamid, verwenden und Lösungsmittel, wie Toluol, Xylol, Tetrahydrofuran, 1,4-Dioxan, Äther, Sulfolan, Dimethylsulfoxid oder Hexamethylphosphorsäuretriamid, einsetzen.

Die als Ausgangsmaterialien dienenden 3-Phenylpyrazolin-5-one sind bekannt oder können nach bekannten Methoden gewonnen werden. Ihre Alkalimetallsalze können mit oder ohne Isolierung mit dem Chlorid, Bromid oder Jodid, insbesondere dem Chlorid oder Bromid, der Formel III umgesetzt werden.

Die Hydrolyse der Ester, Nitrile oder Amide der Formel I kann im sauren oder alkalischen Medium vorgenommen werden. Zur sauren Hydrolyse eignen sich verdünnte Salzsäure oder Schwefelsäure bei Temperaturen von 60 bis 100°C. Geeignete Lösungsmittel sind niedere Alkohole,

Tetrahydrofuran, 1,4-Dioxan, allein oder im Gemisch mit Wasser. Die basische Hydrolyse geschieht vorzugsweise mit wäßrigem Alkali, z.B. Natriumhydroxid, Kaliumhydroxid, Kaliumcarbonat oder Natriumcarbonat bei einer Temperatur zwischen 15 und 100°C, bevorzugt zwischen 40 und 60°C, und liefert das entsprechende Alkalisalz der Carbonsäure der allgemeinen Formel I.

Nach dem unter b) beschriebenen Verfahrensaspekt wird ein Alkalimetallsalz des 3-Phenylpyrazolin-5-ons der Formel II mit einem Haloform, wie Chloroform, Bromoform oder Jodoform, vorzugsweise Chloroform, und einer Carbonylverbindung der Formel IV in Gegenwart eines Alkalihydroxids, vorzugsweise Natriumhydroxid oder Kaliumhydroxid, bei etwa 20 bis 100°C, vorzugsweise 50 bis 70°C, bei Normaldruck oder erhöhtem Druck umgesetzt.

Aus den bei der Umsetzung entstehenden Alkalimetallsalzen können die freien Carbonsäuren durch Mineralsäuren in Freiheit gesetzt werden.

Die vorstehend unter c) bis e) angeführten Verfahrensaspekte des erfindungsgemäßen Verfahrens betreffen Abwandlungen der endständigen Funktion in der Seitenkette einer Verbindung der Formel I.

So hat der Verfahrensaspekt $c_1$) die Umwandlung der Carboxylgruppe in die ggf. substituierte Amidgruppe zum Gegenstand. Die Umwandlung einer Säurefunktion in eine Amidfunktion kann in an sich bekannter Weise direkt durch Umsetzung der freien Säure mit Ammoniak oder primären oder sekundären Alkylaminen bewirkt werden. Es ist von Vorteil, diese Umwandlung zweistufig durchzuführen, indem man die Säure vorerst in ein zur Darstellung von Säureamiden geeignetes reaktives Derivat, z.B. in ein

Säurehalogenid, insbesondere das Säurechlorid, oder auch in ein reaktionsfähiges Säureanhydrid überführt, worauf das Säurechlorid oder Säureanhydrid anschließend in situ mit Ammoniak oder dem Alkylamin umgesetzt wird.

Die Überführung in ein Säurechlorid kann z.B. durch Behandeln mit Phosphorpentachlorid oder Thionylchlorid erfolgen. Die Reaktion wird zweckmäßigerweise ohne Lösungsmittel oder in Gegenwart eines inerten organischen Lösungsmittels, wie chlorierten Kohlenwasserstoffen, z.B. Methylenchlorid oder Chloroform, bei Temperaturen, die in der Nähe der Raumtemperatur liegen, z.B. zwischen -20 und +30°C, durchgeführt. Zur Herstellung des Säureanhydrids setzt man die Säure zweckmäßigerweise mit einem Chlorameisensäurealkylester, z.B. dem Äthylester, um. Die Reaktionstemperatur liegt zweckmäßigerweise zwischen -5°C und Raumtemperatur. Als Reaktionsmedium kann ein inertes Lösungsmittel dienen, z.B. Chloroform, Toluol, Tetrahydrofuran, Dimethylformamid.

Die Umsetzung des gebildeten Säurechlorids oder Säureanhydrids mit Ammoniak oder einem primären oder sekundären Alkylamin erfolgt vorteilhafterweise bei einer Temperatur zwischen -5°C und Raumtemperatur und in einem Lösungsmittel, wie Wasser, Chloroform, Toluol, Tetrahydrofuran, Dimethylformamid und Gemischen derselben. Es kann vorteilhaft sein, das zur Umsetzung benutzte Amin im Überschuß als säurebindendes Reagenz zu verwenden oder die Umsetzung in Gegenwart anderer säurebindender Reagenzien, wie z.B. Kaliumcarbonat, Natriumcarbonat, Natriumhydroxid, Kaliumhydroxid, Triäthylamin oder Pyridin durchzuführen. Im Überschuß eingesetzt, können die Amine zusätzlich als Lösungsmittel dienen.
Nach dem Verfahrensaspekt $c_2$) können die Säuren der Formel V durch nachträgliche Veresterung in an sich bekannter Weise in die entsprechenden Carbonsäureester übergeführt werden.

So können die Ester erhalten werden, indem man die Carbonsäuren der Formel V in dem Alkohol löst, welchen man als Alkoholkomponente im Ester wünscht, und die Umsetzung mit einer Mineralsäure oder auch durch eine organische Säure, z.B. p-Toluolsulfonsäure, katalysiert. Die Temperaturen liegen zwischen Raumtemperatur und dem Siedepunkt des eingesetzten Alkohols, vorzugsweise zwischen 40°C und 60°C. Als Mineralsäuren verwendet man Salzsäure, Schwefelsäure oder Phosphorsäure, vorzugsweise Schwefelsäure oder auch gasförmigen Chlorwasserstoff. Gegebenenfalls können den Reaktionspartnern geeignete inerte Lösungsmittel wie Chloroform, Tetrahydrofuran oder Toluol zugegeben werden.

Man kann die Ester auch dadurch erhalten, daß man die Säuren der Formel V in geeignete Salze, z.B. in die Alkalimetallsalze, wie Lithium-, Kalium- oder Natriumsalze, Ammoniumsalze oder Silbersalze überführt und diese mit Verbindungen der Formel VIII

$$R^6\text{-}X$$

worin $R^6$ einen Alkylrest mit 1 bis 4 C-Atomen, geradkettig oder verzweigt, und X ein Halogenatom aus der Reihe Chlor, Brom oder Jod oder einen reaktiven organischen Säurerest, wie die Tosyloxy- oder Mesyloxygruppe, bedeutet, zwischen 15 und 80°C, vorzugsweise 40 bis 60°C, und ggf. in Gegenwart eines inerten Lösungsmittels, wie Methylenchlorid, Chloroform, Tetrahydrofuran, 1,4-Dioxan, Toluol, Xylol, Dimethylformamid oder Dimethylsulfoxid, umsetzt.

Man kann die Herstellung der Ester aus den Säuren aber auch zweistufig durchführen, indem man Säurehalogenide

(Säurechloride, Säurebromide oder Säurefluoride) oder Anhydride, wie oben beschrieben, aus den Säuren herstellt und diese mit einem Alkalimetallalkoholat der Formel IX

$$R^6\text{-OMe}$$

worin $R^6$ die obengenannte Bedeutung hat und Me für Natrium oder Kalium, vorzugsweise Natrium, steht, in den entsprechenden Alkoholen bei Temperaturen zwischen -10°C und der Siedetemperatur des Lösungsmittels, vorzugsweise zwischen 0 und 25°C, umsetzt.

Nach dem vorstehenden Verfahrensaspekt d) werden Nitrile der Formel VI nach an sich bekannten Methoden zu den entsprechenden primären Amiden der Formel I, worin $R^1$, $R^2$, $R^3$, $R^4$, $R^5$ und A die oben angegebene Bedeutung haben, hydrolysiert. Die Umsetzung kann in Mineralsäuren, z.B. Salzsäure, Schwefelsäure, Phosphorsäure oder auch mit Chlorwasserstoff, in einem geeigneten Lösungsmittel, z.B. niederen Alkanol wie Äthanol, erfolgen, ebenso alkalisch, z.B. mit Natrium- oder Kaliumhydroxid, gegebenenfalls unter Zugabe von Wasserstoffperoxid und in einem geeigneten Lösungsmittel, wie Wasser, 1,4-Dioxan, Chloroform, Toluol oder Gemischen derselben. Die Reaktionstemperaturen liegen zwischen 25 und 100°C, vorzugsweise zwischen 40 und 80°C.

Nach dem vorstehenden Verfahrensaspekt e) können durch Ammonolyse oder Aminolyse von Estern der Formel VII in an sich bekannter Weise durch Behandlung mit Ammoniak oder einem primären bzw. sekundären Alkylamin bei Normaldruck oder bei erhöhtem Druck die entsprechenden Amide hergestellt werden. Man arbeitet dabei zweckmäßigerweise in einem inerten organischen Lösungsmittel, z.B. einem niederen Alkanol, wie Äthanol, einem Kohlenwasserstoff,

wie Benzol, oder einem chlorierten Kohlenwasserstoff, wie Chloroform. Man kann gegebenenfalls auch wäßrige Lösungen von Ammoniak bzw. des Amins einsetzen. Die Reaktionstemperatur liegt in der Regel zwischen Raumtemperatur und etwa 150°C und ist abhängig von dem verwendeten Lösungsmittel. Im Überschuß eingesetzt, können die Amine auch als Lösungsmittel dienen.

Aus dem obigen Verfahrensschema ergibt sich, daß eine Möglichkeit der Herstellung der ggf. substituierten Amide auch ausgehend von den Säuren über die Ester besteht.

Der Verfahrensaspekt f) ist eine alternative Darstellungsmöglichkeit der Ester der Formel VII, in welchen $R^3$ ein Alkyl-, Cycloalkyl- oder Benzylrest ist, aus solchen Estern, in welchen $R^3$ ein Wasserstoffatom ist. Die Umsetzung kann dadurch erfolgen, daß man den Ester in einem geeigneten Lösungsmittel bei Temperaturen zwischen Raumtemperatur und 100°C, vorzugsweise zwischen 60 und 80°C, mit einer starken Base, z.B. einem Alkalialkoholat, wie Natriummethylat, Natriumäthylat oder Kalium-tert.-butylat, einem Alkaliamid, wie Natriumamid oder Lithiumdiisopropylamid, oder einem Alkalihydrid wie Natriumhydrid, zur Reaktion bringt und die Reaktionslösung anschließend mit einer Verbindung $R^3$-X versetzt, wobei $R^3$ die obengenannte Bedeutung hat und X für ein Halogen, vorzugsweise Chlor, Brom und Jod, aber auch für einen reaktiven organischen Säurerest, wie die Tosyloxy- oder Mesyloxygruppe, steht. Geeignete Lösungsmittel sind z.B. Toluol, Xylol, Dimethylformamid oder Diäthylenglycoldimethyläther; mit Vorteil verwendet man Toluol oder Dimethylformamid.

Als pharmakologisch verträgliche Salze der Säuren der Formel I kommen die von Natrium, Ammonium, Calcium

oder Magnesium in Frage, außerdem z.B. die des Morpholins oder Äthanolamins.

Die erfindungsgemäßen Substanzen und ihre Salze weisen wertvolle therapeutische Eigenschaften auf. Dieses läßt sich anhand der folgenden Testergebnisse zeigen.

Beschreibung der pharmakologischen Untersuchungsmethoden

1.) Akute Toxizität
Die akute 7-Tage-Toxizität wurde nach einmaliger Applikation an der weißen, nüchternen NMRI-Maus bestimmt. Die Berechnung der $LD_{50}$-Werte erfolgte über EDV durch eine Probitanalyse (L. Cavalli-Storza, Gustav-Fischer-Verlag, Stuttgart (1964), Grundbegriffe der Biometrie, S.153 ff.).

2.) Tierversuche
An männlichen Sprague-Dawley-Ratten mit diätetischer Hyperlipidämie (orotsäurefreie Handler'sche Diät; S.B. Standerfer und P. Handler, Proc. Soc. exper. Biol. Med. 90, 270 (1955); Zusatz 2 % Cholesterin und 1 % Cholsäure) wurde im Vergleich zu Clofibrat die lipidsenkende Wirkung der Testsubstanz nach der Versuchsanordnung von B. Blank, F.R. Pfeiffer, C.M. Greenberg und J.F. Kerwin, (J. med. Chem. 6, 650 (1963)) untersucht.

Die Testsubstanz wurde über einen Zeitraum von 4 Wochen in Dosierungen von 100 und 200 mg/kg Körpergewicht p.o. täglich per Magensonde in 10 ml einer 1%-igen Traganth-Lösung verabreicht. Clofibrat wurde entsprechend in Dosierungen von 100 und 200 mg/kg gegeben. Die Kontrolltiere erhielten 10 ml der Trägerflüssigkeit Traganth/kg Körpergewicht per Magensonde.

Jede Gruppe bestand aus 20 Tieren; das Gewicht der Leber wurde festgestellt und verglichen.

3.) Klinisch-chemische Untersuchungen

Die Blutentnahme erfolgte im Ätherrausch aus dem retrobulbären Venenplexus.

Die Bestimmung des Gesamt-Cholesterins wurde mit dem Folch-Sperry-Extrakt nach Zöllner und Eberhagen (s. "Untersuchung und Bestimmung der Lipide im Blut", S. 38 und 286, Springer-Verlag, Berlin, 1965) und der Methode nach Zak und Mitarbeitern (s. Am. J. Clin. Path. 24, 1307 (1954)) durchgeführt.

Die Bestimmung der Triglyceride erfolgte mit dem Folch-Sperry-Extrakt nach der Methode von Eggstein und Kreutz (s. Klin. Wschr. 44, 262 (1966)), modifiziert von Schmidt und Dahl (s. J. Klin. Chem. und Klin. Biochem. 6, 151 (1968)).

Statistische Prüfung

Ausgeführt wurden Varianzanalyse und Student-t-Test. Als Signifikanzgrenze diente $p \leq 0,01$.

Als Testsubstanz dient die Verbindung 1,5-Diphenyl-3-(2-aminocarbonylpropyl-2-oxy)-pyrazol (Substanz A).

Die folgende Zusammenstellung zeigt die Wirkung dieser Verbindung in verschiedenen Dosierungen auf die Serumlipide Cholesterin und Triglyceride sowie auf das Lebergewicht. Zum Vergleich ist die Wirkung von Clofibrat (α-(p-Chlorphenoxy)-α-methyl-propionsäureäthylester) in der Tabelle 1 gegenübergestellt.

Tabelle 1

| | Serum Gesamt-Cholesterin mmol/l | Serum Trigly-ceride mmol/l | Leber-gewicht g/100 g |
|---|---|---|---|
| Kontrolle | 15,2 ± 2,4 | 1,04 ± 0,34 | 7,3 ± 0,7 |
| Substanz A* 100 mg/kg | 6,8 ± 2,4 | 0,83 ± 0,20 | 7,2 ± 0,6 |
| Substanz A* 200 mg/kg | 5,5 ± 1,4 | 0,70 ± 0,16 | 7,4 ± 0,7 |
| Clofibrat** 100 mg/kg | 6,9 ± 2,1 | 0,90 ± 0,16 | 8,2 ± 0,9 |
| Clofibrat** 2oo mg/kg | 5,9 ± 1,7 | 0,86 ± 0,66 | 8,2 ± 0,7 |

* $LD_{50}$ p.o. > 1470 mg/kg (i.p. 571)
** $LD_{50}$ p.o. > 1470 mg/kg (i.p. 566)

Aus der Zusammenstellung ist zu erkennen, daß das 1,5-Diphenyl-3-(2-aminocarbonylpropyl-2-oxy)-pyrazol ausgezeichnete lipidsenkende Eigenschaften hat. Gegenüber dem gebräuchlichsten Therapeutikum Clofibrat zeichnet sich diese Verbindung durch eine stärker senkende Wirkung auf die Serumparameter Cholesterin und Triglyceride aus. Ein wesentlicher Vorteil dieser Substanz besteht darin, daß sie keinerlei Veränderungen der Leber, wie Zunahme des Lebergewichtes und Leberzellvergrößerung, hervorruft, während unter der Behandlung mit den bekannten Therapeutika des Clofibrat-Typs Lebervergrößerungen und Leberzellproliferationen auftreten.

Durch die notwendige Langzeitbehandlung von Fettstoffwechselstörungen (Hyperlipoproteinämien) sind die Veränderungen der Leber unerwünschte und nicht ungefährliche Nebenerscheinungen.

Erfindungsgemäße Verbindungen dieses Typs eignen sich aufgrund der lipidsenkenden Wirkung und des nicht schädigenden Einflusses auf die Leber als ausgezeichnete Langzeittherapeutika der Indikation Hyperlipidämien.

4.) Bestimmung der Triglyceridsenkung

Die Verbindungen wurden auf triglyceridsenkende Eigenschaften an der unbehandelten Ratte über einen Zeitraum von 4 Tagen untersucht. Die Substanzen wurden in der Dosierung von 200 mg/kg per Magensonde in 5 ml 1%-iger Traganth-Lösung appliziert. Die Blutentnahme erfolgte im Ätherrausch aus dem retrobulbären Venenplexus. Die Serumtriglyceride wurden nach Eggstein und Kreutz (Klin. Wschr. 44, 262 (1966)) bestimmt. Jede Gruppe bestand aus 10 Tieren. Die statistische Prüfung erfolgte nach dem Student-t-Test. Als Signifikanzgrenze diente $p \leq 0,01$.

Die Triglyceridsenkung durch die untersuchten Substanzen in Prozent gegenüber der Kontrolle ist der Tabelle 2 zu entnehmen.

Toxizitäten der in der Tabelle 2 angeführten Substanzen:

| Substanz Nr. | $LD_{50}$ p.o. mg/kg | $LD_{50}$ i.p. mg/kg |
|---|---|---|
| 1 | >1470 | 684 |
| 2 | >1470 | >100 |
| 3-8 | > 300 | >100 |

Tabelle 2

| Substanzen | Triglycerid-senkung in % |
|---|---|
| 5-Phenyl-3-(2-aminocarbonylpropyl-2-oxy)-pyrazol | 15 |
| 1-Phenyl-5-(4-chlorphenyl)-3-(2-aminocarbonyl-propyl-2-oxy)-pyrazol | 20 |
| 1-Phenyl-5-(3-chlorphenyl)-3-(2-aminocarbonyl-propyl-2-oxy)-pyrazol | 30 |
| 5-(4-Chlorphenyl)-3-(2-aminocarbonylpropyl-2-oxy)-pyrazol | 15 |
| 1-Benzyl-5-(4-chlorphenyl)-3-(2-aminocarbonyl-propyl-2-oxy)-pyrazol | 15 |
| 1-Phenyl-5-(4-chlorphenyl)-3-(2-äthoxycarbonyl-propyl-2-oxy)-pyrazol | 20 |
| 5-Phenyl-3-(2-äthoxycarbonylpropyl-2-oxy)-pyrazol | 25 |
| 1,5-Diphenyl-3-(2-hydroxycarbonylpropyl-2-oxy)-pyrazol | 30 |

Aus Tabelle 2 ist zu ersehen, daß sich diese erfindungsgemäßen Substanzen durch eine selektive Triglyceridsenkung auszeichnen. Die z.Zt. gebräuchlichen Therapeutika (Clofibrat und Nikotinsäure) besitzen diese Selektivität nicht. Damit ergibt sich eine Möglichkeit, endogene wie auch exogene Hypertriglyceridämien mit diesen Verbindungen gezielt und erfolgreich zu behandeln.

Die erfindungsgemäßen Substanzen besitzen auch gute antiallergische Eigenschaften.

So zeigen beispielsweise 5-Phenyl-3-(2-hydroxycarbonylpropyl-2-oxy)-pyrazol und 5-(4-Chlorphenyl)-3-(2-hydroxycarbonylpropyl-2-oxy)-pyrazol und ihre Salze bei geringer Toxizität eine ausgeprägte perorale antianaphylaktische Wirksamkeit, wie an Versuchstieren

durch den PCA-Test (passive cutaneous anaphylaxis reaction) gezeigt werden konnte. Die neuen Verbindungen eignen sich insbesondere aufgrund ihrer oralen antianaphylaktischen Wirksamkeit zur Behandlung von allergischen Reaktionen.

Die Verbindungen der allgemeinen Formel I und ihre Salze lassen sich in bekannter Weise in die üblichen pharmazeutischen Zubereitungsformen, z.B. in Lösungen, Suppositorien, Tabletten, Kapseln oder Dragées, einarbeiten. Die Einzeldosis beträgt für Erwachsene bei oraler Applikation 20 bis 200 mg.

Beispiel I

Tabletten mit 200 mg 1,5-Diphenyl-3-(2-aminocarbonylpropyl-2-oxy)-pyrazol als Wirkstoff.

1 Tablette enthält:

| | | |
|---|---|---|
| Wirkstoff | 200 mg ) | |
| Maisstärke | 40 mg ) | Granulat |
| Amijel | 20 mg ) | |
| Maisstärke | 8 mg ) | |
| Magnesiumstearat | 4 mg ) | Zumischung |
| | 272 mg | |

Herstellungsverfahren: Es werden der Wirkstoff, die Maisstärke und das Amijel (vorgelatinierte Maisstärke) miteinander vermischt und anschließend mit 32 ml destilliertem Wasser pro 200 g Wirkstoff granuliert. Das feuchte Granulat wird durch ein 2,5 mm-Sieb passiert und dann über Nacht bei einer Temperatur von 40°C auf Horden getrocknet. Das getrocknete Granulat wird nun durch ein 2,0 mm-Sieb passiert. Nach dem Vermischen der Maisstärke

und dem Magnesiumstearat wird das Gemisch zu Tabletten mit einem Gewicht von 272 mg verpreßt.

Beispiel II

Tabletten mit 20 mg 5-Phenyl-3-(2-hydroxycarbonylpropyl-2-oxy)-pyrazol, Natrium-Salz, als Wirkstoff.

1 Tablette enthält:

| | | |
|---|---|---|
| Wirkstoff | 20 mg ) | |
| Lactose | 80 mg ) | Granulat |
| Maisstärke | 60 mg ) | |
| Gelatine | 3 mg ) | |
| Magnesiumstearat | 2 mg | Zumischung |
| | 165 mg | |

Herstellungsverfahren: Der Wirkstoff, die Lactose und die Maisstärke werden miteinander vermischt und dann mit einer 7,5%-igen wäßrigen Gelatine-Lösung granuliert. Die feuchte Masse passiert man durch ein 1,6 mm-Sieb und nach dem Trocknen über Nacht bei 40°C auf Horden durch ein 1,0 mm-Sieb. Das Granulat wird mit dem Magnesium-Stearat vermischt und dann zu Tabletten mit einem Gewicht von 165 mg verpreßt.

Die nachstehenden Beispiele dienen zur Erläuterung der Erfindung.

Beispiel 1

47,2 g 2,3-Diphenylpyrazolin-5-on werden in 300 ml Dimethylformamid gelöst und unter Rühren bei 80°C mit 6 g Natriumhydrid (80%-ig) versetzt. Nach 30 min. werden 39 g 2-Brom-2-methylpropionsäureäthylester in 50 ml Dimethylformamid hinzugetropft. Nach 24-stündigem Rühren wird das Lösungsmittel unter vermindertem Druck abgezogen und der Rückstand mit Äthylacetat aufgenommen. Aus der organischen Phase läßt man Reste von unumgesetztem 2,3-Diphenylpyrazolin-5-on auskristallisieren und trennt sie ab. Der nach dem Eindampfen des Lösungsmittels verbleibende ölige Rückstand kristallisiert aus Cyclohexan/Äther. Es werden 45,3 g (64,7 % d.Th.) 1,5-Diphenyl-3-(2-äthoxycarbonylpropyl-2-oxy)-pyrazol mit Fp. 60-61°C erhalten.

Unter entsprechenden Reaktionsbedingungen lassen sich durch Umsetzung von 2-Brom-2-methylpropionsäureäthylester mit

2-Phenyl-3-(4-chlorphenyl)-pyrazolin-5-on
2-Phenyl-3-(3,4-dichlorphenyl)-pyrazolin-5-on
3-Phenylpyrazolin-5-on
3-(4-Chlorphenyl)-pyrazolin-5-on
3-(3,4-Dichlorphenyl)-pyrazolin-5-on
3-(2-Chlorphenyl)-pyrazolin-5-on
3-(4-Trifluormethylphenyl)-pyrazolin-5-on

folgende Verbindungen erhalten:

| | Fp. °C |
|---|---|
| 1-Phenyl-5-(4-chlorphenyl)-3-(2-äthoxycarbonylpropyl-2-oxy)-pyrazol | 49- 50 |
| 1-Phenyl-5-(3,4-dichlorphenyl)-3-(2-äthoxycarbonylpropyl-2-oxy)-pyrazol | 95- 96 |

| | Fp. °C |
|---|---|
| 5-Phenyl-3-(2-äthoxycarbonylpropyl-2-oxy)-pyrazol | 78- 79 |
| 5-(4-Chlorphenyl)-3-(2-äthoxycarbonylpropyl-2-oxy)-pyrazol | 104-105 |
| 5-(3,4-Dichlorphenyl)-3-(2-äthoxycarbonylpropyl-2-oxy)-pyrazol | 118-119 |
| 5-(2-Chlorphenyl)-3-(2-äthoxycarbonylpropyl-2-oxy)-pyrazol. IR: 1744 $cm^{-1}$ (Carbonyl) | Öl |
| 5-(4-Trifluormethylphenyl)-3-(2-äthoxycarbonylpropyl-2-oxy)-pyrazol. IR: 1715 $cm^{-1}$ (Carbonyl) | Öl |

Beispiel 2

35,0 g 1,5-Diphenyl-3-(2-äthoxycarbonylpropyl-2-oxy)-pyrazol werden in 80 ml Äthanol gelöst und mit 12 g Natriumhydroxid in 80 ml Wasser 1 h unter Rückfluß gekocht. Das Äthanol wird weitgehend abdestilliert und der wäßrige Rückstand unter Eiskühlung mit 15%-iger Salzsäure bis auf pH 1 angesäuert. Die ausgefallene Carbonsäure wird abgesaugt und aus Äther/Petroläther umkristallisiert. Es werden 28,8 g (89,4 % d. Th.) 1,5-Diphenyl-3-(2-hydroxycarbonylpropyl-2-oxy)-pyrazol mit Fp. 149-150°C erhalten.

Unter Einhaltung entsprechender Reaktionsbedingungen erhält man aus

5-Phenyl-3-(2-äthoxycarbonylpropyl-2-oxy)-pyrazol
5-(2-Chlorphenyl)-3-(2-äthoxycarbonylpropyl-2-oxy)-pyrazol
5-(4-Chlorphenyl)-3-(2-äthoxycarbonylpropyl-2-oxy)-pyrazol
5-(3,4-Dichlorphenyl)-3-(2-äthoxycarbonylpropyl-2-oxy)-pyrazol
5-(4-Trifluormethylphenyl)-3-(2-äthoxycarbonylpropyl-2-oxy)-pyrazol

1-Äthyl-5-phenyl-3-(2-äthoxycarbonylpropyl-2-oxy)-pyrazol

1-Benzyl-5-phenyl-3-(2-äthoxycarbonylpropyl-2-oxy)-pyrazol

1-Benzyl-5-(4-chlorphenyl)-3-(2-äthoxycarbonylpropyl-2-oxy)-pyrazol

1-Benzyl-5-(3,4-dichlorphenyl)-3-(2-äthoxycarbonylpropyl-2-oxy)-pyrazol

folgende Verbindungen:

| | Fp. °C |
|---|---|
| 5-Phenyl-3-(2-hydroxycarbonylpropyl-2-oxy)-pyrazol | 161-162 |
| 5-(2-Chlorphenyl)-3-(2-hydroxycarbonylpropyl-2-oxy)-pyrazol | 172-174 |
| 5-(4-Chlorphenyl)-3-(2-hydroxycarbonylpropyl-2-oxy)-pyrazol | 190 |
| 5-(3,4-Dichlorphenyl)-3-(2-hydroxycarbonylpropyl-2-oxy)-pyrazol | 193-194 |
| 5-(4-Trifluormethylphenyl)-3-(2-hydroxycarbonylpropyl-2-oxy)-pyrazol | 199 |
| 1-Äthyl-5-phenyl-3-(2-hydroxycarbonylpropyl-2-oxy)-pyrazol. IR: 1705 $cm^{-1}$(Carbonyl) | Öl |
| 1-Benzyl-5-phenyl-3-(2-hydroxycarbonylpropyl-2-oxy)-pyrazol | 143-145 |
| 1-Benzyl-5-(4-chlorphenyl)-3-(2-hydroxycarbonylpropyl-2-oxy)-pyrazol. IR: 1695 $cm^{-1}$(Carbonyl) | Öl |
| 1-Benzyl-5-(3,4-dichlorphenyl)-3-(2-hydroxycarbonylpropyl-2-oxy)-pyrazol.IR:1702$cm^{-1}$(Carbonyl) | Öl |

Beispiel 3

23,6 g 2,3-Diphenylpyrazolin-5-on und 22,5 g Natriumhydroxid werden in 125 ml Aceton und 10 ml Chloroform suspendiert und unter Rühren 2 h auf 60°C erwärmt. Das Lösungsmittel wird darauf bei vermindertem Druck abgezogen, der feste Rückstand in Wasser gelöst und unter Eiskühlung mit 20%-iger Salzsäure auf pH 2 gebracht. Die feste Carbonsäure wird abgesaugt und aus Äther/Petroläther umkristallisiert. Der Schmelzpunkt des 1,5-Diphenyl-3-(2-hydroxycarbonylpropyl-2-oxy)-pyrazol beträgt 149-150°C, die Ausbeute ist 22,1 g (68,7 % d.Th.). Unter entsprechenden Reaktionsbedingungen erhält man aus 47,2 g 2,3-Diphenylpyrazolin-5-on, 22,5 g Natriumhydroxid, 250 ml Methyläthylketon und 20 ml Chloroform 34,5 g (51,4 % d.Th.) 1,5-Diphenyl-3-(2-hydroxycarbonylbutyl-2-oxy)-pyrazol mit Fp. 122-124°C bzw. aus

13,5 g 2-Phenyl-3-(3-chlorphenyl)-pyrazolin-5-on, 11,3 g Natriumhydroxid, 65 ml Aceton und 5 ml Chloroform 11,7 g (65,8 % d.Th.) 1-Phenyl-5-(3-chlorphenyl)-3-(2-hydroxycarbonylpropyl-2-oxy)-pyrazol mit Fp. 190°C.

<u>Beispiel 4</u>

16,1 g 1,5-Diphenyl-3-(2-hydroxycarbonylpropyl-2-oxy)-pyrazol werden in 300 ml Dichlormethan gelöst und unter Eiskühlung mit 5,1 g Triäthylamin und 5,5 g Chlorameisensäureäthylester in 20 ml Dichlormethan versetzt. Diese Lösung wird unter Rühren in 100 ml konzentrierte wäßrige Ammoniaklösung eingegossen. Nach 1 h wird die organische Phase abgetrennt und die wäßrige Phase mehrmals mit Methylenchlorid extrahiert. Die vereinigten organischen Phasen werden über Natriumsulfat getrocknet und das Lösungsmittel unter vermindertem Druck abgezogen. Der verbleibende feste Rückstand wird aus Cyclohexan/Methylenchlorid umkristallisiert. Es werden 12,0 g (74,9 % d.Th.) 1,5-Diphenyl-3-(2-aminocarbonylpropyl-2-oxy)-pyrazol mit Fp. 125-126°C erhalten.

Entsprechend werden aus

1,5-Diphenyl-3-(1-hydroxycarbonyl-2-methylpropyl-1-oxy)-pyrazol
1,5-Diphenyl-3-(2-hydroxycarbonylbutyl-2-oxy)-pyrazol
1-Phenyl-5-(3-chlorphenyl)-3-(2-hydroxycarbonylpropyl-2-oxy)-pyrazol
1-Phenyl-5-(4-chlorphenyl)-3-(2-hydroxycarbonylpropyl-2-oxy)-pyrazol
1-Phenyl-5-(3,4-dichlorphenyl)-3-(2-hydroxycarbonylpropyl-2-oxy)-pyrazol
1-Phenyl-5-(4-trifluormethylphenyl)-3-(2-hydroxycarbonylpropyl-2-oxy)-pyrazol
5-Phenyl-3-(2-hydroxycarbonylpropyl-2-oxy)-pyrazol
5-(4-Chlorphenyl)3-(2-hydroxycarbonylpropyl-2-oxy)-pyrazol
1-Äthyl-5-phenyl-3-(2-hydroxycarbonylpropyl-2-oxy)-pyrazol
1-Benzyl-5-phenyl-3-(2-hydroxycarbonylpropyl-2-oxy)-pyrazol
1-Benzyl-5-(4-chlorphenyl)-3-(2-hydroxycarbonylpropyl-2-oxy)-pyrazol
1-Benzyl-5-(3,4-dichlorphenyl)-3-(2-hydroxycarbonylpropyl-2-oxy)-pyrazol
1,5-Diphenyl-3-hydroxycarbonylmethoxy-pyrazol

folgende Verbindungen erhalten:

| | Fp. °C |
|---|---|
| 1,5-Diphenyl-3-(1-aminocarbonyl-2-methylpropyl-1-oxy)-pyrazol | 166-168 |
| 1,5-Diphenyl-3-(2-aminocarbonylbutyl-2-oxy)-pyrazol | 111-113 |
| 1-Phenyl-5-(3-chlorphenyl)-3-(2-aminocarbonylpropyl-2-oxy)-pyrazol | 113-114 |
| 1-Phenyl-5-(4-chlorphenyl)-3-(2-aminocarbonylpropyl-2-oxy)-pyrazol | 128-130 |
| 1-Phenyl-5-(3,4-dichlorphenyl)-3-(2-aminocarbonylpropyl-2-oxy)-pyrazol | 128-130 |

| | Fp. °C |
|---|---|
| 1-Phenyl-5-(4-trifluormethylphenyl)-3-(2-aminocarbonylpropyl-2-oxy)-pyrazol | 118 |
| 5-Phenyl-3-(2-aminocarbonylpropyl-2-oxy)-pyrazol | 150-151 |
| 5-(4-Chlorphenyl)-3-(2-aminocarbonylpropyl-2-oxy)-pyrazol | 160-161 |
| 1-Äthyl-5-phenyl-3-(2-aminocarbonylpropyl-2-oxy)-pyrazol | 75- 76 |
| 1-Benzyl-5-phenyl-3-(2-aminocarbonylpropyl-2-oxy)-pyrazol | 98 |
| 1-Benzyl-5-(4-chlorphenyl)-3-(2-aminocarbonylpropyl-2-oxy)-pyrazol | 116 |
| 1-Benzyl-5-(3,4-dichlorphenyl)-3-(2-aminocarbonylpropyl-2-oxy)-pyrazol | 110-111 |
| 1,5-Diphenyl-3-aminocarbonylmethoxy-pyrazol | 137-138 |

Beispiel 5

25,8 g 1,5-Diphenyl-3-(2-hydroxycarbonylpropyl-2-oxy)-pyrazol werden in 500 ml Dichlormethan gelöst und bei 0°C mit 8,1 g Triäthylamin und 8,7 g Chlorameisensäureäthylester in 30 ml Dichlormethan versetzt. Die Lösung wird in 150 ml 40%-ige wäßrige Methylamin-Lösung gegossen und 1 h gerührt. Nach Aufarbeitung werden 18,1 g (67,4 % d.Th.) 1,5-Diphenyl-3-(2-N-methylaminocarbonylpropyl-2-oxy)-pyrazol mit Fp. 151°C erhalten.

Durch Eingießen des obigen Anhydrids in eine Lösung von Diäthylamin in Dichlormethan erhält man 1,5-Diphenyl-3-(2-N,N-diäthylaminocarbonylpropyl-2-oxy)-pyrazol als Öl. IR: 1695 $cm^{-1}$ (Carbonyl).

Beispiel 6

Zu 41,1 g 5-Phenyl-3-(2-äthoxycarbonylpropyl-2-oxy)-pyrazol, gelöst in 250 ml Toluol, werden unter Rühren bei 80°C 4,5 g Natriumhydrid (80%-ig) gegeben. Anschließend

werden 16,4 g Äthylbromid in 50 ml Toluol zugetropft und die Lösung 24 h gerührt. Nach Aufarbeitung werden 34,6 g (76,4 % d.Th.) 1-Äthyl-5-phenyl-3-(2-äthoxycarbonylpropyl-2-oxy)-pyrazol als Öl erhalten. IR: 1740cm$^{-1}$ (Carbonyl).

Entsprechend werden aus 54,8 g 5-Phenyl-3-(2-äthoxycarbonylpropyl-2-oxy)-pyrazol und 25,3 g Benzylchlorid 48,6 g (66,7 % d.Th.) 1-Benzyl-5-phenyl-3-(2-äthoxycarbonylpropyl-2-oxy)-pyrazol als Öl erhalten. IR: 1738cm$^{-1}$ (Carbonyl).

Aus 46,3 g 5-(4-Chlorphenyl)-3-(2-äthoxycarbonylpropyl-2-oxy)-pyrazol und 19,0 g Benzylchlorid werden 34,6 g (57,9 % d.Th.) 1-Benzyl-5-(4-chlorphenyl)-3-(2-äthoxycarbonylpropyl-2-oxy)-pyrazol als Öl erhalten. IR: 1730 cm$^{-1}$(Carbonyl).

Aus 34,3 g 5-(3,4-Dichlorphenyl)-3-(2-äthoxycarbonylpropyl-2-oxy)-pyrazol und 12,7 g Benzylchlorid erhält man 24,6 g (56,7 % d.Th.) 1-Benzyl-5-(3,4-dichlorphenyl)-3-(2-äthoxycarbonylpropyl-2-oxy)-pyrazol als Öl. IR: 1735 cm$^{-1}$(Carbonyl).

Aus 13,7 g 5-Phenyl-3-(2-äthoxycarbonylpropyl-2-oxy)-pyrazol und 8,2 g Cyclohexylbromid werden 8,4 g (47,2 % d.Th.) 1-Cyclohexyl-5-phenyl-3-(2-äthoxycarbonylpropyl-2-oxy)-pyrazol als Öl erhalten. IR: 1746 cm$^{-1}$(Carbonyl).

Aus 13,7 g 5-Phenyl-3-(2-äthoxycarbonylpropyl-2-oxy)-pyrazol und 6,9 g Isobutylbromid werden 7,2 g 1-Isobutyl-5-phenyl-3-(2-äthoxycarbonylpropyl-2-oxy)-pyrazol als Öl erhalten. IR: 1738 cm$^{-1}$(Carbonyl).

Beispiel 7

Zu 70,8 g 2,3-Diphenylpyrazolin-5-on in 500 ml Dimethylformamid werden unter Rühren bei 80°C 9 g Natriumhydrid (80%-ig) gegeben. Nach Zugabe von 23 g Chloracetonitril in 50 ml Dimethylformamid wird weitere 5 h auf 80°C erwärmt. Nach Aufarbeitung und Reinigung erhält man 59,9 g

(72,6 % d.Th.) 1,5-Diphenyl-3-cyanomethoxy-pyrazol als Öl. IR: 2235 $cm^{-1}$ (Nitril).

Entsprechend werden 23,6 g 2,3-Diphenylpyrazolin-5-on in 150 ml Dimethylformamid unter Rühren bei 80°C mit 3 g Natriumhydrid (80%-ig) versetzt. Anschließend werden 14,8 g 2-Brom-2-methylpropionitril in 30 ml Dimethylformamid hinzugetropft und die Reaktionslösung 15 h gerührt. Nach Aufarbeitung und chromatographischer Reinigung werden 18,5 g (60 % d.Th.) 1,5-Diphenyl-3-(2-cyanopropyl-2-oxy)-pyrazol als Öl erhalten. IR: 2225 $cm^{-1}$ (Nitril).

Beispiel 8

27,5 g 1,5-Diphenyl-3-cyanomethoxy-pyrazol werden mit 150 ml einer 15%-igen methanolischen Kaliumhydroxidlösung 1 h unter Rückfluß gekocht. Das Methanol wird unter vermindertem Druck abgezogen, der Rückstand in Wasser gelöst und mit 15%-iger Salzsäure bis auf pH 2 angesäuert. Die ausgefallene Säure wird abgesaugt und aus Isopropylalkohol umkristallisiert. Es werden 24,3 g (82,7 % d.Th.) 1,5-Diphenyl-3-hydroxycarbonylmethoxy-pyrazol mit Fp. 173-175°C erhalten.

Beispiel 9

8,1 g 1,5-Diphenyl-3-(2-hydroxycarbonylpropyl-2-oxy)-pyrazol in 200 ml 2-Propanol werden unter Eiskühlung mit 3 ml konzentrierter Schwefelsäure versetzt und 24 h auf 40°C erwärmt. Nach Abziehen des Lösungsmittels wird der Rückstand mit 10%-iger wäßriger Natriumhydroxidlösung alkalisch gemacht und der Ester mit Äthylacetat extrahiert. Der aus der organischen Phase gewonnene Rückstand wird in n-Pentan umkristallisiert. Es werden 7,6 g (83,1 % d.Th.) 1,5-Diphenyl-3-(2-isopropyloxycarbonylpropyl-2-oxy)-pyrazol mit Fp. 84-85°C erhalten.

0007019

Entsprechend wird aus 5-Phenyl-3-(2-hydroxycarbonylpropyl-2-oxy)-pyrazol und Methanol das 5-Phenyl-3-(2-methoxycarbonylpropyl-2-oxy)-pyrazol erhalten, Fp. 111-112°C.

Beispiel 10

12,3 g 5-Phenyl-3-(2-hydroxycarbonylpropyl-2-oxy)-pyrazol in 100 ml 2-Propanol werden mit einer Lösung von 2 g Natriumhydroxid in 5 ml Wasser versetzt. Die Lösung wird auf etwa die Hälfte ihres Volumens eingeengt und bis zum Beginn der Kristallisation mit Äther versetzt. Es werden 13,4 g (quantitativ) des Natriumsalzes des 5-Phenyl-3-(2-hydroxycarbonylpropyl-2-oxy)-pyrazols mit Fp. 278-280°C erhalten.

Auf entsprechende Weise werden hergestellt:

| | Fp. °C |
|---|---|
| die Natriumsalze von | |
| 5-(4-Chlorphenyl)-3-(2-hydroxycarbonylpropyl-2-oxy)-pyrazol | 297-299 |
| 5-(2-Chlorphenyl)-3-(2-hydroxycarbonylpropyl-2-oxy)-pyrazol | 209-211 |
| 5-(2-Trifluormethylphenyl)-3-(2-hydroxycarbonylpropyl-2-oxy)-pyrazol | 275-276 |
| 5-Phenyl-3-hydroxycarbonylmethoxy-pyrazol | 280-283 |
| 1,5-Diphenyl-3-(2-hydroxycarbonylpropyl-2-oxy)-pyrazol | 322-325 |
| 1,5-Diphenyl-3-(2-hydroxycarbonylmethoxy)-pyrazol | 269-272 |

In entsprechender Weise werden aus 7 g 5-(4-Chlorphenyl)-3-(2-hydroxycarbonylpropyl-2-oxy)-pyrazol in 50 ml 2-Propanol und 50 ml 25%-iger wäßriger Ammoniaklösung 7,4 g (quantitativ) des Ammoniumsalzes des 5-(4-Chlorphenyl)-3-(2-hydroxycarbonylpropyl-2-oxy)-pyrazols erhalten. Die Substanz zersetzt sich unter $NH_3$-Entwicklung bei 133°C.

Die freiwerdende Säure schmilzt bei 190°C.

Beispiel 11

17,5 g 1,5-Diphenyl-3-(2-äthoxycarbonylpropyl-2-oxy)-pyrazol werden in 250 ml konzentrierter wäßriger Ammoniaklösung 3 h unter Rückfluß gekocht. Das Lösungsmittel wird unter vermindertem Druck bis zur Trockne eingeengt. Es verbleibt ein öliger Rückstand, der aus Methylenchlorid/Äther kristallisiert. Es werden 6,8 g (42,4 % d.Th.) 1,5-Diphenyl-3-(2-aminocarbonylpropyl-2-oxy)-pyrazol mit Fp. 125-126°C erhalten.

Beispiel 12

18 g 1,5-Diphenyl-3-(2-cyanopropyl-2-oxy)-pyrazol in 120 ml Chloroform werden mit 4,8 g Natriumhydroxid in 50 ml Wasser versetzt. Man erwärmt auf 50°C, tropft 30 ml 30%-iges wäßriges Wasserstoffperoxid so hinzu, daß die Temperatur 55°C nicht übersteigt und rührt 8 h. Nach Zugabe weiterer 20 ml 30%-igen Wasserstoffperoxids rührt man noch 24 h bei 60°C. Nach dem Abkühlen wird auf Eiswasser gegossen, mit Chloroform extrahiert und die organische Phase mit Natriumsulfitlösung peroxidfrei gewaschen. Der nach Abdestillieren des Lösungsmittels verbleibende ölige Rückstand kristallisiert nach Zugabe von Äther. Das erhaltene 1,5-Diphenyl-3-(2-aminocarbonylpropyl-2-oxy)-pyrazol schmilzt bei 125-126°C. Die Ausbeute beträgt 12,9 g (67 % d. Th.).

Beispiel 13

4,7 g 2,3-Diphenylpyrazolin-5-on werden in 50 ml Dimethylformamid gelöst und unter Rühren bei 80°C mit 600 mg Natriumhydrid (80%-ig) versetzt. Nach 15 min. werden 4,5g 2-Brom-2-methylpropionsäure-N,N-diäthylamid in 15 ml Dimethylformamid hinzugetropft. Nach 18-stündigem Rühren wird wie üblich aufgearbeitet. Nach chromatographischer Reinigung erhält man 5,2 g (60% d.Th.) 1,5-Diphenyl-3-(2-N,N,-diäthylaminocarbonylpropyl-2-oxy)-pyrazol als Öl. IR: 1695 $cm^{-1}$ (Carbonyl).

## Patentansprüche

1. Neue 5-Phenylpyrazol-Derivate der Formel I

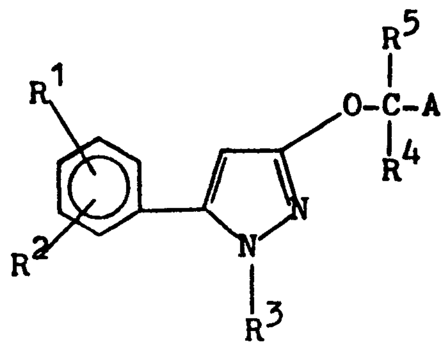

worin $R^1$ und $R^2$ gleich und verschieden sein können und ein Wasserstoff- oder Chloratom oder eine Trifluormethylgruppe bedeuten, $R^3$ ein Wasserstoffatom, ein Phenyl-, Benzyl- oder Alkylrest mit 1 bis 6 C-Atomen, welcher geradkettig, verzweigt oder cyclisch sein kann, $R^4$ ein Wasserstoffatom oder Methylrest, $R^5$ ein Alkylrest mit 1 bis 4 C-Atomen, geradkettig oder verzweigt, und A Carboxyl, Alkoxycarbonyl, worin der Alkoxyrest 1 bis 4 C-Atome hat und geradkettig oder verzweigt ist, Cyano, Aminocarbonyl, Monoalkylaminocarbonyl oder Dialkylaminocarbonyl mit Alkylresten mit 1 bis 3 C-Atomen ist sowie die pharmakologisch verträglichen Salze der Säuren.

2. 1-A-5-B-3-(2-äthoxycarbonylpropyl-2-oxy)-pyrazol, in welchem A Phenyl und B Phenyl, 4-Chlorphenyl oder 3,4-Dichlorphenyl sind oder A Äthyl und B Phenyl sind oder A Benzyl und B Phenyl, 4-Chlorphenyl oder 3,4-Dichlorphenyl sind oder A Cyclohexyl oder Isobutyl und B Phenyl sind oder A ein Wasserstoffatom und B Phenyl, 2-Chlorphenyl, 4-chlorphenyl, 3,4-Dichlorphenyl oder 4-Trifluormethylphenyl sind.

3. 1,5-Diphenyl-3-(2-isopropyloxycarbonylpropyl-2-oxy)-pyrazol.

4. 5-Phenyl-3-(2-methoxycarbonylpropyl-2-oxy)-pyrazol.

5. 1-A-5-B-3-(2-hydroxycarbonylpropyl-2-oxy)-pyrazol, in welchem A Phenyl und B Phenyl oder 3-Chlorphenyl sind, A Äthyl und B Phenyl sind, A Benzyl und B Phenyl, 4-Chlorphenyl oder 3,4-Dichlorphenyl sind, oder A ein Wasserstoffatom und B Phenyl, 2-Chlorphenyl, 4-Chlorphenyl, 3,4-Dichlorphenyl oder 4-Trifluormethylphenyl sind.

6. 1,5-Diphenyl-3-C-pyrazol, in welchem C Hydroxycarbonylmethoxy oder 2-Hydroxycarbonylbutyl-2-oxy ist.

7. 1-A-5-B-3-(2-aminocarbonylpropyl-2-oxy)-pyrazol, in welchem A Phenyl und B Phenyl, 3-Chlorphenyl, 4-Chlorphenyl, 3,4-Dichlorphenyl, 4-Trifluormethylphenyl sind oder A Äthyl und B Phenyl sind, oder A Benzyl und B Phenyl, 4-Chlorphenyl oder 3,4-Dichlorphenyl sind oder A ein Wasserstoffatom und B Phenyl oder 4-Chlorphenyl sind.

8. 1,5-Diphenyl-3-C-pyrazol, in welchem C Aminocarbonylmethoxy, 1-Aminocarbonyl-2-methylpropyl-2-oxy oder 2-Aminocarbonylbutyl-2-oxy, 2-N-Methylaminocarbonylpropyl-2-oxy oder 2-N,N-Diäthylaminocarbonylpropyl-2-oxy ist.

9. 1,5-Diphenyl-3-C-pyrazol, in welchem C Cyanomethoxy oder 2-Cyanopropyl-2-oxy ist.

10. Verfahren zur Herstellung der 5-Phenylpyrazol-Derivate der Formel I

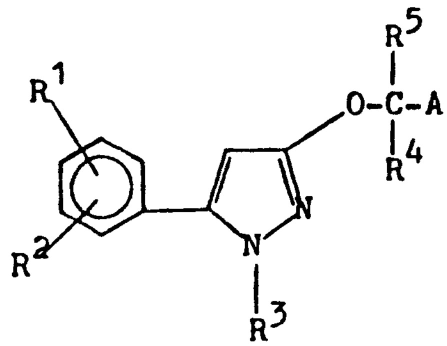

worin $R^1$ und $R^2$ gleich und verschieden sein können und ein Wasserstoff- oder Chloratom oder eine Trifluormethylgruppe bedeuten, $R^3$ ein Wasserstoffatom, ein Phenyl-, Benzyl- oder Alkylrest mit 1 bis 6 C-Atomen, welcher geradkettig, verzweigt oder cyclisch sein kann, $R^4$ ein Wasserstoffatom oder Methylrest, $R^5$ ein Alkylrest mit 1 bis 4 C-Atomen, geradkettig oder verzweigt, und A Carboxyl, Alkoxycarbonyl, worin der Alkoxyrest 1 bis 4 C-Atome hat und geradkettig oder verzweigt ist, Cyano, Aminocarbonyl, Monoalkylaminocarbonyl oder Dialkylaminocarbonyl mit Alkylresten mit 1 bis 3 C-Atomen ist sowie die pharmakologisch verträglichen Salze der Säuren, dadurch gekennzeichnet, daß man

a) ein Alkalimetallsalz des 3-Phenylpyrazolin-5-on der Formel II

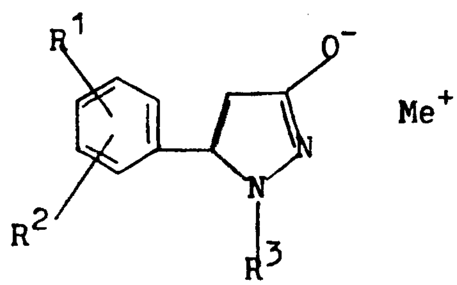

worin $R^1$, $R^2$ und $R^3$ die obengenannte Bedeutung haben und $Me^+$ ein Alkalimetallion ist, mit einer Verbindung der Formel III

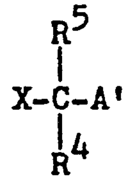

worin $R^4$ und $R^5$ die obengenannte Bedeutung haben, A' eine Alkoxycarbonyl-, eine Cyano- oder eine ggf. substituierte Aminocarbonylgruppe und X ein Halogenatom, vorzugsweise Chlor oder Brom, ist, zu einer Verbindung der Formel I umsetzt, worin $R^1$, $R^2$, $R^3$, $R^4$ und $R^5$ die obengenannte Bedeutung haben und A eine Cyano-, eine Alkoxycarbonyl- oder eine ggf. substituierte Aminocarbonylgruppe ist, gewünschtenfalls ein Säurederivat zur freien Säure oder zu deren physiologisch verträglichem Salz hydrolysiert, oder daß man

b) ein Alkalimetallsalz des 3-Phenylpyrazolin-5-on der Formel II

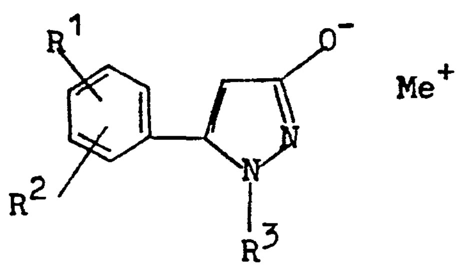

worin $R^1$, $R^2$ und $R^3$ die obengenannte Bedeutung haben und $Me^+$ ein Alkalimetallion ist, mit einem Haloform und einer Carbonylverbindung der Formel IV

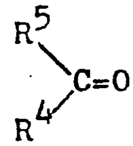

worin $R^4$ und $R^5$ die obengenannte Bedeutung haben, in Gegenwart eines Alkalihydroxids zu einem Alkalisalz einer Verbindung der Formel I umsetzt, in welcher $R^1$, $R^2$, $R^3$, $R^4$ und $R^5$ die obengenannte Bedeutung haben und A eine Carboxylgruppe ist und ggf. das Alkalimetallsalz zur freien Säure hydrolysiert, oder daß man

c) eine Säure der Formel V

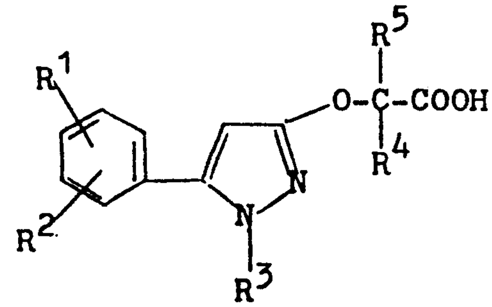

in welcher $R^1$, $R^2$, $R^3$, $R^4$ und $R^5$ die obengenannte Bedeutung haben,

$c_1$) in das entsprechende Amid umwandelt, dessen Amidfunktion durch Alkyl mono- oder disubstituiert sein kann, oder

$c_2$) in den entsprechenden Ester der Formel I umwandelt, oder daß man

d) ein Nitril der Formel VI

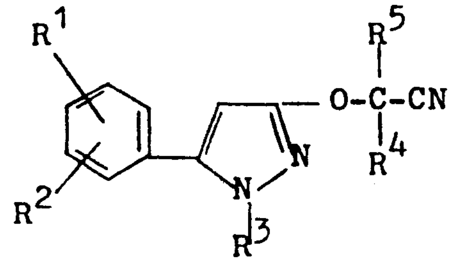

in welcher $R^1$, $R^2$, $R^3$, $R^4$ und $R^5$ die obengenannte Bedeutung haben, in das entsprechende Amid umwandelt, oder daß man

e) einen Ester der Formel VII

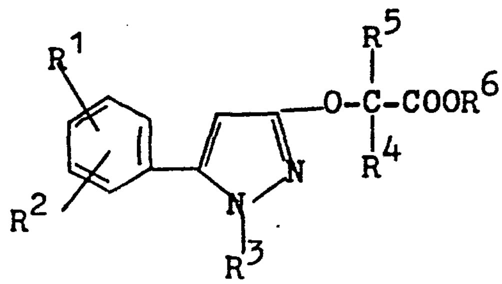

worin $R^1$, $R^2$, $R^3$, $R^4$ und $R^5$ die obengenannte Bedeutung haben und $R^6$ eine niedere Alkylgruppe ist, in das entsprechende Amid umwandelt, dessen Amidfunktion durch Alkyl mono- oder disubstituiert sein kann, oder daß man

f) einen Ester der Formel VII, worin $R^1$, $R^2$, $R^4$, $R^5$ und $R^6$ die obengenannte Bedeutung haben und $R^3$ ein Wasserstoffatom ist, in den entsprechenden Ester umwandelt, in welchem $R^3$ Alkyl, Cycloalkyl oder Benzyl ist.

11. Arzneimittel, bestehend aus einer oder mehreren Verbindungen gemäß Anspruch 1 und üblichen Träger- und Hilfssubstanzen.

Europäisches Patentamt

# EUROPÄISCHER RECHERCHENBERICHT

Nummer der Anmeldung
EP 79 102 022.5

| EINSCHLÄGIGE DOKUMENTE | | | KLASSIFIKATION DER ANMELDUNG (Int Cl.3) |
|---|---|---|---|
| Kategorie | Kennzeichnung des Dokuments mit Angabe, soweit erforderlich, der maßgeblichen Teile | betrifft Anspruch | |
| X | DD - A - 118 638 (H. DORN et al.)<br>* Ansprüche 1, 2, Seite 3, Zeilen 5 bis 15 *<br>-- | 1,10, 11 | C 07 D 231/20<br>A 61 K 31/415<br>C 07 D 231/22 |
| P | DE - A1 - 2 809 183 (AKADEMIE DER WISSENSCHAFTEN DER DDR)<br>* Ansprüche 2, 9, 10 * | 10,11 | |
| P | & DD - A - 135 487<br>-- | | RECHERCHIERTE SACHGEBIETE (Int. Cl.3) |
| A | DE - A1 - 2 347 015 (SCHERING)<br>---- | | A 61 K 31/415<br>C 07 D 231/20<br>C 07 D 231/22 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

✓ Der vorliegende Recherchenbericht wurde für alle Patentansprüche erstellt.

| Recherchenort | Abschlußdatum der Recherche | Prüfer |
|---|---|---|
| Berlin | 19-10-1979 | FROELICH |